# EUROPEAN PATENT SPECIFICATION

(11) **EP 2 205 253 B1**
(45) Date of publication and mention of the grant of the patent: **08.08.2012**
(21) Application number: 08788816.0
(22) Date of filing: 27.08.2008
(51) Int. Cl.: A61K 35/74, A61P 1/00, A61P 3/00, A61P 5/00, A61P 9/00, A61P 17/00, A61P 25/00, A61P 27/00, A61P 31/00, A61K 35/00, A61P 37/00

(54) **COMPOSITION AND METHOD INHIBITING INFLAMMATION**
ENTZÜNDUNGSHEMMENDE ZUSAMMENSETZUNG UND VERFAHREN
COMPOSITION ET PROCÉDÉ INHIBANT L'INFLAMMATION

(30) Priority: 27.08.2007 HU 0700552
(43) Date of publication of application: 14.07.2010
(73) Proprietor: Feher, Janos, 1021 Budapest (HU)
(72) Inventor: Feher, Janos, 1021 Budapest (HU)
(74) Representative: Somfai, Eva
(86) International application number: PCT/HU2008/000098
(87) International publication number: WO 2009/027753

(56) References cited:
- EP-A- 0 135 820
- WO-A-00/23069
- WO-A-03/033681
- WO-A-2006/118988
- US-A1- 2002 044 957
- US-A1- 2006 228 403
- LIN M-Y ET AL.,: "Inhibition of lipid peroxidation by Lactobacillus and Bifidobacterium longum" J. AGRIC. FOOD CHEM., [Online] vol. 47, no. 9, 1999, pages 3661-3664, XP002512654 Retrieved from the Internet: URL:http://pubs.acs.org/doi/abs/10.1021/jf 981235l>
- DATABASE WPI Week 200126 Thomson Scientific, London, GB; AN 2001-252979 XP002512660 & JP 2001 048796 A (ADVANCE CO LTD) 20 February 2001 (2001-02-20)
- DATABASE WPI 22 March 2004 (2004-03-22), Thomson Scientific, London, GB; AN 2004-485439 XP002512661 & KR 2004 024 740 A ((MAEI-N) MAEIL DAIRY IND CO LTD) 22 March 2004 (2004-03-22)
- DATABASE BIOSIS [Online] BIOSCIENCES INFORMATION SERVICE, PHILADELPHIA, PA, US; April 2006 (2006-04), CAROL MONICA ET AL: "Viable bacteria versus DNA extracts as anti-inflammatory agents in Crohn's disease" XP002512657 Database accession no. PREV200600502748 & GASTROENTEROLOGY, vol. 130, no. 4, Suppl. 2, April 2006 (2006-04), page A128, DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085
- DATABASE MEDLINE [Online] US NATIONAL LIBRARY OF MEDICINE (NLM), BETHESDA, MD, US; October 1987 (1987-10), SAITO H ET AL: "Protective and therapeutic efficacy of Lactobacillus casei against experimental murine infections due to Mycobacterium fortuitum complex." XP002512658 Database accession no. NLM3130460 & JOURNAL OF GENERAL MICROBIOLOGY OCT 1987, vol. 133, no. 10, October 1987 (1987-10), pages 2843-2851, ISSN: 0022-1287
- SASHIHARA T ET AL: "An analysis of the effectiveness of heat-killed lactic acid bacteria in alleviating allergic diseases" JOURNAL OF DAIRY SCIENCE, vol. 89, no. 8, August 2006 (2006-08), pages 2846-2855, XP002512655 ISSN: 0022-0302
- DATABASE BIOSIS [Online] BIOSCIENCES INFORMATION SERVICE, PHILADELPHIA, PA, US; 1998, SUZUKI KEIJI ET AL: "Pathogenesis of Lactobacillus casei cell wall extract-induced cardiovasculitis" XP002512659 Database accession no. PREV199900244715 & ACTA HISTOCHEMICA ET CYTOCHEMICA, vol. 31, no. 6, 1998, pages 465-473, ISSN: 0044-5991
- SEGAWA SHUICHI ET AL: "Oral administration of heat-killed lactobacillus brevis SBC8803 ameliorates the development of dermatitis and inhibits immunoglobulin E production in atopic dermatitis model NC/Nga mice" BIOLOGICAL & PHARMACEUTICAL BULLETIN, vol. 31, no. 5, May 2008 (2008-05), pages 884-889, XP002512656 ISSN: 0918-6158

## Description

This invention is related to compositions inhibiting inflammation and to methods for treating inflammation of humans and mammals with compositions according to the invention. By way of introducing their active principles into the plasma cellular membrane redox system (PMRS) these compositions inhibit generation of lipid peroxides from membrane lipids which represent the earliest event of the inflammatory chain or cascade. Thus, these compositions may be used to prevent, attenuate and/or inhibit inflammation and inflammation-related diseases. The basis of the invention is a method for preventing, treating or attenuating inflammatory diseases by way of inhibiting lipid peroxidation and subsequent elementary inflammation through ensuring the presence of the following biologically active ingredient in the PMRS of cells involved in inflammation, prepared as a stable lipid emulsion,
said emulsion comprising
a) as biologically active ingredient a combination consisting of:
   i) at least one killed probiotic and
   ii) at least one omega 3 FA and
   iii) vitamin E and
b) as formulation additives water and a pharmaceutically acceptable emulgator
   where the particle size in the lipid emulsion is 0,001 to 100 microns,
   where the mass ratio of omega 3 fatty acid to water is 100 : 1 to 100000
   or as
   an aqueous solution
   comprising
   a) as biologically active ingredient a combination for elementary inhibition of inflammation consisting of:
      i) at least one killed probiotic and
      ii) water-soluble salt or ester of at least i one omega 3 FA and
      iii) water-soluble ester of natural semi-synthetic or synthetic vitamin E, and
   b) as pharmaceutically acceptable formulation additives water and
      water-soluble formulation additives selected from
      excipients, vehicles, preservatives and colorants
      and characterized by the following mass ratios of ingredients:
      i) Omega 3 fatty acids: vitamin E = 100 : 1 to 100;
      ii) Omega 3 fatty acids: probiotics = 100 : 0,01 to 100
by introducing a composition comprising any of the missing ingredients.

Some terms used frequently in this specification are defined as follows (if not specifically stated otherwise) :
*Anaerobic glycolysis or Fermentation*: a process of energy production in a cell under anaerobic conditions (with no oxygen required) . Sugars are the common substrate of fermentation, and typical examples of fermentation products are *lactic acid*, and *hydrogen*.
Arachidonic acid (AA) : omega 6 type PUFA, precursor of prostaglandines and leukotrienes.
*Composition for Elementary Inhibition of Inflammation*
Combination according to the present invention of compounds probiotics, omega 3 FA and vitamin E as each of them should come from exogeneous sources
*Cyclooxygenase (COX)*: enzyme transforming AA into prostaglandines
*Elementary Inflammation (definition of the inventor):*
formation of lipid peroxides from membrane lipids, which represents the initial event in generating inflammation ("inflammatory cascade"). This is always associated with altered membrane functions unless specifically indicated.
*Inflammation:* is a reaction of human and animal organism to noxious agent or stimuli characterized by rubor (redness), color (heat), tumor (swelling), dolor (pain), function laesa (loss of function). Prostaglandines and leukotriennes are the principal proinflammatory cytokines responsible for inflammation.
*Lipoxygenase (LOX)* :enzyme transforming AA into leukotrienes.
*Metabolic antioxidant process*: electrons from anaerobic glycolysis through NAD(P)H and NADH are transported into PMRS, and these electrons prevent generation of lipid peroxides as well as regenerate oxidized CoQ, vitamin E and omega 3 PA in this order.
*Method for Elementary Inhibition of Inflammation:* methods for inhibiting generation of lipid peroxides in the PM, (synonymously Inhibition of Elementary Inflammation); definition first used by the inventor *Mitochondrium* a membrane-enclosed organelle, found in most eukaryotic cells, except red blood cells. They generate most of the cell's supply of ATP used as a source of chemical energy.
*NOX (NADH oxidase)* : located at the outer side of the plasma membrane, transfers electrons from reduced CoQ to the molecules on the outer side of plasma membrane (ascorbat, proteins, oxygen)
*Omega 6 fatty acid*: a family of PUFA, which have in common, a carbon-carbon double bond in the ω-6 position
*Omega-3 fatty acids*: a family of PUFA, which have in common a carbon-carbon double bond in the ω-3 position
*Organelles:* submicroscopic structures of cell having specialized functions. These are mitochondria, endoplasmic reticulum, Golgy apparatus, lysosomes, and peroxisomes.
*Phospholipase A2:* located at the inner side of plasma membrane, activated by lipid peroxides, and causes release of arachidonic acid from membrane lipids.
*Plasma Membrane Redox System (PMRS)*: an electron transport system composed of (i) NADH oxidase (NOX) (ii) ubiquinone (CoQ) (iii) Ubiquinone reductase.
*Polyunsaturated Fatty Acids (PUFA)*: Fatty acids containing more than one carbon-carbon double bond.
*Probiotic* microorganisms or their derivates, which confer a beneficial health effect on the host organism. These are *Lactobacilli*, *Bifidobacteria*, *Saccharomyces boulardi*, *Saccharomyces cerevisiae, Monascus pupurea*.
*Ubiquinone (CoQ)*: an intramembrane redoxactive molecule. Its reduced form is called ubiquinol; its oxidized form is called ubiquinone.
*Ubiquinone reductase:* located at the inner side of the plasma membrane it transfers electrons from NAD(P)H and NADH to CoQ.
Abbreviations used in this specification are explained as follows:

| | |
|---|---|
| AA | arachidonic acid [20:4 (n-6)] |
| ALA, | α-linolenic acid [18:3 (n-3)] |
| BB | Bifidobacterium |
| CoQ | ubiquinone, Coenzyme Q, |
| CoQ10 | ubiquinone 10, Coenzyme Q10, |
| DHA | docosahexaenoic acid [22:6 (n-3)] |
| EPA | eicosapentaenoic acid [20:5 (n-3)] |
| LA | linoleic acid [18:3(n-6] |
| LB | Lactobacillus or cell free (killed) extract of Lactobacillus in water (suspension or solution) |
| LTA | lipoteichoic acid |
| NAD(P)H) | Nicotinamide Adenine Dinucleotide Phosphate H |
| NADH | Nicotinamide Adenine Dinucleotide H |
| NOX | NADH oxydase |
| NSAID | Non-Steroid Anti-Inflammatory Drug |
| PM | Plasma Membrane |
| PMRS | Plasma Membrane Redox System |
| PUFA | PolyUnsaturated Fatty Acid |
| ROS | Reactive Oxygen Species ("free radicals") |
| SAID | Steroid Anti-Inflammatory Drug |

It is well known that all human and animal cells are separated from their surroundings by a specially organized membrane, the so-called plasma membrane (PM). From a biochemical point of view, the basal structure of PM is a lipid bilayer, composed mostly of phospholipids, while cholesterol is present in minor quantities. Phospholipid molecules arranged in the PM so that the phosphoric acid "polar heads" face towards the membrane's internal and external surface while the fatty acid chains, "apolar tails", face each other and thus form the membrane's internal hydrophobic region. Further component of PM are transmebrane protein, peripheral proteins, glicolipids and glycoproteins. The organelles found inside the cells have a similar membrane structure.

Membrane lipids are fundamentally responsible for the functioning of PM. The quantity and ratio of phospholipid - cholesterol, the unsaturated - saturated fatty acids, and the omega 3 - omega 6 fatty acids are characteristics of PM functionality. It is generally accepted that the lipid composition of membranes effects their functioning in at least 6 different ways, namely they modify (i) the membrane's fluidity, (ii) the membrane's permeability (the functioning of the ion-channels), (iii) the activity of the enzymes connected to the membranes, (iv) the density and the affinity of the membrane receptors, (v) the release and activity of the neurotransmitters, and (vi) the release of the proinflammatory cytokines

In this aspect the polyunsaturated fatty acids (PUFA), first of all the members of omega 3 and omega 6 fatty acid (FA) families, have a particularly important role. Precursors of PUFA, alpha-linolenic acid, (ALA) (ω 3) or [18:3 (n-3)] and linoleic acid (LA) (ω 6) or [18:3 (n-6)] come exclusively from meals, since the human body does not have the enzymes necessary to produce them. Therefore the proper quantity and quality of PUFA intake in the daily diet is crucial to replenish even the physiological PUFA loss, thus to maintain the PM normal functioning. The PUPA loss gets worse with certain diseases, so the PUFA intake must be increased.

It is known that because of eating habits in the industrialized countries, today's diets do not contain the necessary omega 3 FA and the ratio of omega 6 is higher than optimal. A reduced omega 3 FA content in the cell membranes, respectively an altered omega 3 - omega 6 ratio has been found in inflammatory and degenerative diseases and tumors, and in certain congenital diseases, so it has been linked to the development of these diseases. It has also been found, that addition of omega 3 FA, or of their natural precursors to the daily diet, lead to an increased omega 3 FA content in the cell membranes, a decreased occurrence of the above listed diseases and a better recovery. The recommended daily allowance for DHA + EPA amounts to 650 mg.

Vitamin E and the CoQ molecules are embedded into the membrane lipids. They have a very important biological role in the maintenance of the normal membrane structure and functioning. Vitamin E is known as a fat-soluble vitamin. There are 8 known forms of vitamin E: α-, β-, γ-, and δ -tocopherols contain saturated phytyl side chains and α-, β-, γ-, and δ d-tocotrienols have 3 double bonds in the side chain. The a -tocopherol molecule is the most potent. It is a 6-hydroxychroman derivative with methyl groups in position 2,5,7, and 8 and a phytyl side chain attached at carbon 2. The chroman ring is the redox-active part, while the phytyl side chain binds it to the PM. Due to its lipophilic nature, vitamin E accumulates in cellular membranes. The major function of vitamin E is to act as a natural antioxidant by scavenging free radicals and molecular oxygen. Vitamin E is important for preventing peroxidation of PUPA in membranes. It comes exclusively from the diet. The recommended daily allowance is 10 mg.

It is further known that CoQ is a fat soluble, redox-active molecule, that has two parts: (i) a quinon ring, which is able to pick-up and release 2 electrons, and (ii) a hydrophobic isopren side-chain connected to it, which locks the molecule in the hydrophobic zone of the PM. In humans, this side-chain has 10 subunits (this is what the 10 refers to in CoQ10). CoQ10 can be found in all cell membranes, that suggests an important biological role. It has been also found in the membranes of bacteria indicating that it had been developed early on during evolution and it has a fundamental role in the antioxidant defense mechanism. In a normal circumstance the body covers its CoQ10 needs with endogenous biosynthesis. This biochemical process is tightly connected to the cholesterol synthesis, In earlier studies; supplementing the daily diet with CoQ10 favorably influenced the inflammatory and degenerative diseases, the tumors and the course of some inherited diseases, too. Addition of omega 3 FA strengthened the effect of the CoQ10. This quality of CoQ10 has been credited exclusively to its effects on the mitochondrial metabolisms, more specifically to its effects on the electron transport chain, which is considered in its totality as an "anti-apoptotic" effect (Biochim Biophys Acta. 2004;1660:171-199). The recommended daily allowance for CoQ10 is not determined.

Recent studies described a molecular organization denominated Plasma Membrane Redox System (PMRS) in the PM. A current concept assigned a central role to the CoQ10 in the functioning of this redox system. According to this concept CoQ10 takes electrons from the cytosolic NADH and NAD(P)H and gets reduced, and then transfers these electrons to the ascorbat or thiol-containing molecules reside on the external leaflet of the PM. Both steeps of this transmembrane electron transport are catalized by specific enzymes (J. Exp. Biol. 2000; 2031513-1521).

Our concept extended this theory at two points assigning active role to the omega 3 FA and vitamin E of the PM and to the cytosolic anaerobic glycolysis.

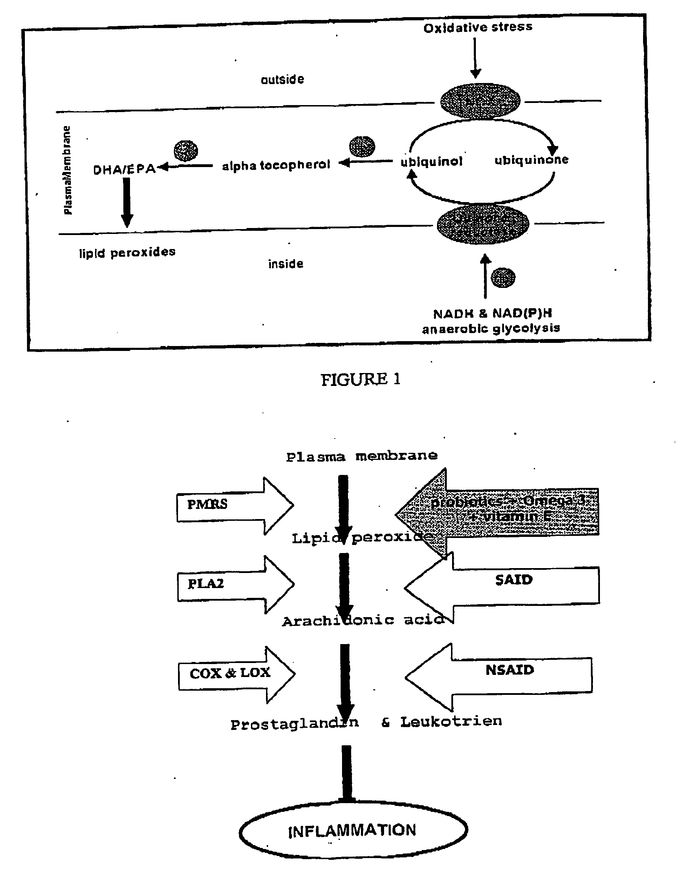

The flow chart represented in Figure 1 schematically summarizes the molecular mechanism of PMRS according to our concept.

This flow-chart summarizes the following functions of the PMRS:
1. *Transmembrane electron transport (orthodox function*):
   delivery of electrons *from* cytosolic NADH and NAD (P) H to PM surface molecules (ascorbates, proteins, molecular oxygen),
2. *Intramembrane electron transport (new function)* :
   delivery of electrons to the intramembrane alpha-tocopherol and omega 3 FA for maintaining or restoring adequate saturation of PM lipids,
3. *Anaerobic glycolysis (new function)* is the principal source of electrons for both transmembrane and intramembrane electron transports

An oxidative stress to membrane causes peroxidation of omega 3 FA. A loss of one or more double bounds results in conformational changes in fatty acid chains, which' may have two consequences: (i) modification of the transmembrane functions, and (ii) initiation of inflammation. As these two functional changes are two consequences of the same processes, we introduced a new term "*elementary inflammation*", which covers both aspects of membrane dysfunction

Ad (i) It is generally accepted that transmembrane proteins and peripheral proteins perform most of the membrane functions. Recent scientific findings showed that amino acid composition of membrane proteins and fatty acid composition of membrane lipids should be matched for normal functioning. Proper function of membrane proteins is conditioned by well-defined lipid environment ("lipid-shell"), and any change in lipid shell results in modification of membrane protein functions. (Biochim Biophys Acta. 2008; 1778:1545-75).

Ad (ii) *The forming of lipid peroxides means a starting point for the beginning of inflammatory processes*. The lipid peroxides activate the phospholipase A2 enzyme, which results in the release of arachidonic acid (AA) from the membrane phospholipids. From the AA, with the help of cyclooxigenase enzyme (COX) prostaglandines are formed, or with the help of lipoxigenase (LOX) leukotrienes are formed. Molecules with many and very diverse biological effects belong to both groups, including the pro- inflammatory molecules.

From the two main groups of anti-inflammatory medications used today, steroids inhibit the AA production with blocking of phospholipase A2, while non-steroids inhibit forming of prostaglandines and leukotrienes with the blocking of COX and LOX. Mechanisms and treatment possibilities including the present invention are summarized in the flow sheet of Figure 2.

**Table 1 EXPLANATION OF THE FIGURES**

| Number | Title | Explanation |
|---|---|---|
| Figure 1 | Plasma Membrane Redox System | Summarizes schematically the molecular mechanism of Plasma Membrane Redox System according to our concept. |
| Figure 2 | Mechanism and Treatment of Inflammation | Summarizes schematically mechanisms and treatment possibilities using anti-inflammatory medications, Including the present invention. |

It is generally accepted that inflammation is the body's coordinated form of defense against the internal and external influences, and it has a key role in maintaining the homeostasis of the cells and organism.

In case of *immunogenic* inflammation, a form of white blood cells, the monocyta or macrophage or dendritic cells have an important role in the detection of the foreign substance and in the production of the so-called pro-inflammatory *cytokines* responsible for creating inflammation.

In case of neurogenic inflammation, the sensory nerves sense the foreign influences, which leeds to the production of pro-inflammatory *neuropeptides*.

These two mechanisms always occur mixed together. This explains the well-known fact, that inflammation is always accompanied by pain, while pain can cause inflammation. Therefore the reduction of inflammation alleviates the pain, and the soothing of pain lessens the inflammation.

In most cases though, the inflammatory reaction can be so strong, that independently from the original reason, the inflammation itself causes damage to the body. Inflammation results in increased metabolism, which leads to the release of an extreme quantity of cell- and tissue-damaging reactive oxygen species (ROS). This explains why the reduction of inflammation goes together with the improvement of the diseased condition, and with the decrease of the damages.

Thanks to the findings obtained in the last decade, more and more date indicate the role of inflammation not merely in infections, allergies and (auto) immune diseases, but also in degenerative diseases characterized by cell loss (apoptosis) and in tumors characterized by cell proliferation where inflammation precedes or causes the degenerative disease or the tumor.

Some main groups of diseases where inflammation has been found to play a pathological role representing potential targets of treatment of the present invention:
- Infections: in bacterial and viral infections, and in the inflammations of the mucus membranes that come with them, like f.e. conjunctivitis, gingivitis, bronchitis, gastritis, vaginitis, and cystitis
- Allergies, like f.e. bronchial asthma, dermatitis, allergic conjunctivitis, hay fever, etc.
- *Autoimmune diseases,* like f.e. rheumatoid arthritis, juvenile (type 1) diabetes, Crohn's disease, ulcerative colitis, psoriasis, lupus erythematosus, sclerosis multiplex. etc.
- *Age related degenerative diseases* like f.e. Alzheimer's, dementia, Parkinson's disease, amyotropic lateral sclerosis, age related macular degeneration, glaucoma, cataract, otosclerosis, osteoporosis, arthrosis, muscle atrophy, hair loss (baldness), etc.
- Neuropsychiatric diseases, like f.e. schizophrenia, depression, anxiety, panic disorder, etc
- *Metabolic syndrome and related diseases,* that includes obesity, type II diabetes, high blood pressure, high blood fat, and fatty liver. atheriosclerosis and its consequences like f.e. coronary disease, stroke, etc.
- *Neovascular diseases*, diabetic retinopathy, wet type AMD, proliferative retinopathy of premature infants.
- Soft tissue damages, like trying sport performances (marathon), sport injuries, contusions, large burns and frost-bites, post-operative inflammations, etc.
- *Tumors,* like f.e. benign and malign tumors of the prostate, and of the cervix, breast cancer, lung cancer, intestinal cancer, lymphoma, etc.

There are several drawbacks of the currently used SAID and NSAID treatments. Both groups have serious side effects that occasionally can be fatal. Furthermore, neither of them influences significantly the natural course of degenerative diseases and tumors. It is also well known that omega 3 FA have a slight anti-inflammatory effect through modifying generation of pro-inflammatory cytokines. Thus they act in the advanced phase of the inflammation cascade. Substances causing the end of the inflammation, also result from omega 3 FA.

Numerous publications in the international literature, widely used compositions in clinical practice, and several patents described the biological and pharmacological influence of omega 3 9A, vitamin E and CoQ either separately or in combination. Here we mention some of therm.

It is known that a combination of omega 3 FA, vitamin E and CoQ has been suggested to treat dysfunctions of mitochondrial metabolism, called mitochondriopathies (EP 1123093). This was based on an observation that omega 3 FA enhances incorporation of CoQ into mitochondrial membranes, where CoQ plays an essential role in the mitochondrial metabolism. Similarly: improvement of mitochondrial metabolism was suggested by a combination containing omega 3 FA, vitamin E, CoQ and L-carnitine or acetyl-1-carnitine (European application 05014812.1).

According to a human dietary study vitamin E and CoQ increased EPA plasma levels, also when fish oil was added to the diet. In some persons decreased AA levels were found. These authors supposed that it makes less favorable conditions for generation of cytokines and thus may have NSAID-like effects (J. of Nutritional & Envir. Med.,1998;8:25-34).

There is also known a food supplement containing omega 3 FA and CoQ with carotenoids, bioflavonoids, water-soluble vitamins and metal ions (USP 6,579,544).

It is also known that lipid emulsions containing soy oil and/or fish oil may be used for enteral or parenteral nutrition for critically ill patients. When fish oil was added to the composition the nutritive effects were accompanied with some decrease of inflammation (Proc. Nutr. Soc., 2006, 264-277).

USP 2002 044957 discloses a nutritional supplement composition comprising a protein source with at least 50% weight whey protein, a lipid source (having an omega 3 to 6 fatty acid ratio of 5:1 to 10:1), a carbohydrate source, vitamin E and vitamin C. In addition, it may incorporate probiotic microorganisms in powdered form, but there is no indication that the microorganisms have to be killed. The composition is used in the manufacture of a functional food or medicament, and it is indicated for convalescing patients recovering from illness or surgery, those with limited appetite such as the elderly, children or anorexic patients as well as for the treatment of oxidative stress or inflammation conditions.

For treating dysfunction of vascular endothelium some food supplements containing combinations of omega 3 FA, CoQ and sugar-amines, such as glucosamine, galactosamine, were suggested in tablets or powder (USP 6,930,099).

The above known combinations also have the significant drawback that the incorporation of these substances is a spontaneous and slow process. Thus their therapeutic effect is casual or low and it develops slowly.

Probiotics - as defined by the Food and Agricultural Organization (FAO) of the United Nations - are "live microorganisms administered in adequate amounts which confer a beneficial health effect on the host." Earlier, probiotics have been used orally as foods, or food supplements, or locally and their physiological benefits were attributed to their *immuno-modulating effects* such as inhibiting colonization of pathogens on the gastrointestinal or uro-genital mucous membranes, and enhancing phagocytic activity of monocytes, macrophages and dendritic cells, stimulating T cell differentiation, and enhancing secretion of immunoglobolin A (IgA). (WO 01/37862 A3 and USP 20080175685 A1 July 24, 2008). This immuno-mudulating effects of both live and killed probiotics were attributed to their cell-wall component, lipoteichoic acid (LTA), an endotoxin of Gram positive bacteria (Proc Natl Acad Sci USA. 2005; 102 (29) :

### Brief summary of the invention

It is an object of the present invention to provide compositions for preventing, treating or attenuating inflammatory diseases by the use of a combination of cell-free extract of killed probiotics and omega 3 FA and vitamin E, or (iii) a combination of cell-free extract of killed probiotics and omega 3 FA and vitamin E and further pharmacologically acceptable active substances, prepared as stable lipid microemulsions or as aqueous solutions.

It is another object of the invention to provide methods for preventing, treating or attenuating inflammatory diseases by the way inhibiting lipid peroxidation and subsequent elementary inflammation through introducing the active ingredients of these compositions into the PMRS of the targeted cells.

Yet it is another object of the invention to provide methods for preventing, treating or attenuating inflammatory diseases by the use of above compositions in lipid emulsion or in water solution formulated for enteral, parenteral and topical use.

It is a further object of the invention to provide the use of the above compositions and methods for preventing, treating or attenuating inflammation related to infectious, immune, degenerative and neoplastic diseases.

### Detailed description of the invention

One scientific basis of our invention is our original observation according to which the PMRS, the surrounding omega 3 FA and vitamin E, as well as the cytoplasmic anaerobic glycolysis, form a *functional unit,* which plays an essential role for survival and adaptation of human and animal cells in both normal conditions and in diseases. Dysfunctions of this unit manifest as *elementary inflammation, which is* the earliest event of inflammation and subsequent inflammatory diseases. Thus, the PMRS represent a new therapeutic target for preventing, treating or attenuating inflammation and inflammation related diseases. The present invention describes compositions and methods suitable to reach these objectives.

The introduction of probiotisc in the treatment regime of inflammation and related diseases represent an original approach. It comes from our observation that exogenous probiotics stimulate the *anaerobic glycolysis* of host cells, which produce more NADH and NAD(P)H to release more electrons into the PMRS for maintaining or restoring adequate redox state of PMRS. This metabolic *antioxidant*/*anti-inflammatory* effect comes from the cytoplasmic fraction of probiotics. It is well known that probiotics are internalized by phagocytes (macrophages, monocytes, endothelial cells, microglia), which are primarily involved in the inflammation, in this way genetic material (DNA, RNA) encoding the anaerobic glycolysis is transported into the host cells, and incorporated into the host genome enhancing their own anaerobic metabolism. This *horizontal gene transfer* is well know in gene-research. It should be kept in mind that genes encoding anaerobic glycolysis are highly conserved during the evolution, thus these genes from probiotics are compatible with those of the host cells. In this context, introduction a cytoplasmic extract of probiotics into human and animal organism works as a "*metabogenic vaccine*" which stimulate anaerobic metabolism, in contrast to the currently used "*immunogenic vaccines*" which use substances for generating immune responses.

Neither of the previous patents or scientific works mentioned that *anaerobic metabolism of probiotics* might be responsible for any beneficial effects of them either in connection with their immunostimulant effects or independently from those. Furthermore, no prior art described probiotic effects on elementary inflammation or on one of the features of elementary inflammation either in animal or human organism. Consequently, these properties of probiotics have never been applied in any treatment regime. Thus, the discovery that anaerobic metabolism of probiotics prevents elementary inflammation is certainly a novelty and it is a key scientific support to this invention.

In the composition the killed probiotic may be full or partial extract of Lactobacillus, and/or Bifidobacterium, and/or Saccharomyces cerevisie or the combination thereof, and/or full or partial extract of biologically acceptable anaerobic bacteria.

In the composition the active ingredients may be the cytoplasmic fraction of killed probiotics, or the nucleotide components (DNA, RNA) of probiotics, or combination thereof, or these.ingredients may also derived from the genetic modification or from the full or partial synthesis of probiotics' DNA and/or RNA

Preferred probiotics are killed *Lactobacillus acidophilus, L. casei, L. plantarum, L. reuteri, L. rhamnosus, L. GG. Z. bulgaricus, L. bifidus, L. caucasicus. L. brevis, L. cellobiosus, L. crispatus, L. curvatus, L. fermentum, L. gasseri, L. johnsonii. L. salivarus; and*/*or Bifidobacterium animalis subsp. lactis, B. bifidum, B. breve, B. infantis, B. longum, B. adolescentis, B. animalis, B. thermophilum, B. lactis, and*/*or Lactococcus lactis (formerly known as Streptococcus lactis), Streptococcus thermophilus, Bacillus coagulans, and*/*or* Enterococcus faecalis, Enterococcus faecium, *and*/*or Saccharomyces boulardii, saccharomyces cerevisiae. Monascus purpureus*

Favorably the quantity of probiotics is 0,01-1000 mg/dose, most preferably 0,1-10mg/dose. This quantity was determined by the protein content of probiotic suspension.

This invention is supported by another original observations that addition of omega 3 FA and vitamin E to probiotics surprisingly enhances the anti-inflammatory effects. This synergy comes primarily from the restoration of PM's omega 3 FA and vitamin E composition and less importantly from the simple addition of anti-inflammatory effects of probiotics and omega 3 FA.. We have mentioned before that PM may contain low levels of these substances duo to either low dietary uptake or chronic environmental influences (smoking, toxins). In these conditions, addition of omega 3 FA to probiotics is essential for obtaining adequate therapeutic effects. No prior art is known on this synergy of killed probiotics, omega 3 FA and vitamin E.

An embodiment of this invention is a composition for elementary inhibition of inflammation in human or mammal organism by inhibition of lipid peroxidation through introducing the following compounds into the cells involved in the inflammation,
a) combination of ingredients for elementary inhibition of inflammation:
   - extract of killed probiotics, and
   - omega 3 FA and
   - vitamin E, and
b) pharmaceutically acceptable excipients, vehicles, preservatives and colorants prepared as stable lipid micro-emulsions or as aqueous solutions.

The composition according to the invention may comprise the omega 3 FA in the form of pharmacologically identical natural form or source of ALA, and/or EPA and/or DHA, and/or ester thereof, preferable an ethyl-ester or trigliceride, and/or omega 3 containing phospholopids preferably phosphatidylinositol, phosphatidylcholine phosphate-dylethanolamine, phosphatidylserine, and sphingomyelin or combinations thereof. The vitamin E may be pure α-tocopherol and/or β-, γ-, or δ-tocopherol, α-, β-, γ-, or δ-tocotrienol and/or their natural, semi-synthetic or synthetic esters.

In the composition the omega 3 FA may be present in form of its precursor such as fish oil. This may be prepared from any part of see-fish preferably from salmon, cod-liver. Also vegetable oil preferably from linseed, rape seed, grape pips and/or the oil derived directly from micro-algae or any other marine living organisms can be used.

Favourably the quantity of omega 3 FA or their ester is 100-1500mg/dose, most preferably 250-750mg/dose.

Favorably the quantity of vitamin E or its ester is 5-500mg/dose, most preferably 15-100mg/dose.

It was also mentioned, that ubiquinone is synthesized endogeneously to cover the body's need. However, in certain conditions it may be insufficient (for example, in use of cholesterol lowering statins) and exogeneous supplement is needed for improving functions of PMRS.

A further embodiment of this invention is a composition for elementary inhibition of inflammation in human or mammal organism by inhibition of lipid peroxidation through introducing the following compounds into the cells involved in the inflammation,
a) combination of ingredients for elementary inhibition of inflammation:
   - extract of killed probiotics, and
   - omega 3 FA and
   - vitamin E, and
   - ubiquinone, and
b) pharmaceutically acceptable excipients, vehicles, preservatives and colorants prepared as stable lipid micro-emulsions or as aqueous solutions.

Favorably the quantity of ubiquinone or its water-soluble derivates is 10-500mg/dose, most preferably 20-100mg/dose.

Compositions according to this invention may further contain pharmacologically compatible ingredients such as vitamin A, B, C, D, F, K and/or corticosteroids, sex-steroids, metal ions (sodium, calcium, magnesium, potassium, phosphor, zinc, iron, selenium) and L-carnitine, aminocarnitines, alpha-lipoic acid, glutathion, essential amino acids, bioflavonoids, polyphenols, terpenes, alkaloides (berberine), volatile oils. amino acids, antibiotics, glycosamino-glycans (hyaluronic acid, chondroitin-sulphate, heparin, heparin-sulphate).

The compositions may further contain formulation additives such as excipients, vehicles, preservatives and colorants selected according to the actual ingredients and the intended method of administration.

According to our own studies a formulation for *limpid emulsion* (oil in water or water in oil) significantly increased the efficacy of our combinations. The vascular endothelial cells and white blood cells may directly incorporate active substances by receptor mediated processes and/or phagocytosis. Both play an essential role in generating inflammation. Furthermore, in this way a lower dosage may be effective. Finally, in the clinical practice, by parenteral lipid emulsion, we may avoid all drawbacks of enteral administration, first of all malabsorption of lipids typical and common in biliary dysfunctions. Topical administration of lipid emulsion in form of solution, gel or ointments also increases the bioavailability of these compounds. They can reach directly the involved inflammatory cells on the conjunctiva, on the gastrointestinal or uro-genital mucous membranes or on the skin surface.

According to our invention, lipid emulsion similarly to other known and used compositions is a mixture of water (with or without additional water-soluble substances) and oil (with liposuluble substances) and they form a stable emulsion specifically in the presence of a suitable emulsifier. Our invention comprises both "water in oil" and "oil in water" types depending on the mass ratio of water and oil and the intended use form.

A further and particularly important embodiment of our invention is the formulation of *lipid emulsion* for delivery of the active ingredients. These lipid emulsions contain
- A combination of the above listed ingredients
- Water forming a stable lipid emulsion in which the mass ratio of omega 3 FA or their esters to water is 100:1-100000; and
- A pharmacologically acceptable emulsifier.
In this context probiotics, omega 3 FA, and vitamin E represent all the previously listed forms ingredients in which these active ingredients are present (precursors or derivates) without repeating them again.

Emulsifiers in lipid emulsions favorably are either of egg- or soy-lecithin, bile, bile acids, Tween^{®} MT, polyvinyl alcohol.

The particle size in lipid emulsion is 0,001 to 100 microns, preferably 0,01 to 5 micron.

Further ingredients/excipients of lipid emulsions (conservants, pH and osmolarity stabilizers) are identical with those of the commercially available products, but the quality of parenteral administration should be guaranteed (see USP 5760020)

According to our invention lipid emulsions are particularly favorable for parenteral (intravenous, intramuscular, intradermal, intraarticular, intraocular, intralesional, para-lesional, subcutaneous) application. In these cases the lipid emulsion may contain further specific pharmacologically acceptable excipients. This formulation is very suitable for rapid delivery of active ingredients for reaching prompt effects on the sites of inflammation. In certain cases this speed may be life saving.

Lipid emulsions may also be formulated for enteral (oral, rectal) transdermal, and nasal administration. In these cases they may contain further, pharmacologically compatible substances, excipients habitually used for similar formulations. The oral administration is particularly important.

For the aqueous phase of lipid emulsion either distilled water or a physiological salt solution is used most frequently.

Further possible formulations of lipid emulsion may be for direct administration in *topical medication,* such as eye-drope, gel, spray, ointment, solutions for lotion, which contain further known and pharmacologically and chemically compatible auxiliary substances and vehicles

The lipid emulsion may also be formulated for oral use in *soft capsules.* This *composition* - for enteral use - may contain all the previously listed preferred substances. Particularly favorably are compositions containing full extract of killed lactobacillus or that of a saccharomyces, omega 3 FA and vitamin E.

Another embodiment of this invention is a water-*soluble composition* for preventing or inhibiting elementary inflammation. These contain as active ingredients either of the following combinations:
- A water-soluble full or partial cell-free extract of at least one of the probiotics.
- A water-soluble salt or ester of at least one of the omega 3 FA, and water-soluble ester of natural, semi-synthetic or synthetic vitamin E,
- Optionally a water soluble salt or derivate of the above listed ingredients
- A pharmacologically acceptable water solution.
In this context probiotics, omega 3 FA, vitamin E and the optional water soluble salt or derivate of the above listed ingredients represent all the previously listed forms ingredients in which these active ingredients are present (precursors or derivates) without repeating them again.

Such compositions may further contain water-soluble excipients, vehicles, preservatives and colorants.

According to our invention a water-soluble composition as described above preferably for parenteral use (via endovenous infusion or injection, subcutaneous or intramuscular injection); and for enteral use (via oral, intra-gastric, transrectal); and for topical use (lotions, eye-drops, nasal-drops, ear-drops, spray, cream, gel) and for liposome encapsulated delivery.

Compositions and combinations according to our invention are for both human and veterinary use either in enteral or parenteral or topical forms of administration.

Further embodiment of this invention is the use of above described compositions in lipid emulsion or in water-solution for preventing, treating and attenuating the following diseases:
- *Inflammation and sepsis*, particularly those which are accompanied with bacterial, viral and fungal infections
- *Inflammation of mucous membranes* such as conjunctivitis, periodotitis, oesophagitis, reflux disease, gastritis, enteritis, colitis, cholecystitis, cystitis, pyelo-nephritis, sinusitis, bronchitis, vaginitis, prostatitis, and related diseases thereof such as hepatitis, cirrhosis, nephritis, pleuritis, fibrosis cystica;
- *Autoimmune inflammation,* such as rheumatoid arthritis, juvenile (type I) diabetes, Chron's disease, colitis ulcerosa, psoriasis, lupus erithematous, multiplex sclerosis.
- *Allergic inflammation* such as asthma, atopic dermatitis, hay-fever, allergic conjunctivitis, allergic rhinitis,
- *Neuropsychiatric diseases,* such as schizophrenia, depression, anxiety, panic-disease.
- *Metabolic syndrome* (dyslipidemia, hypertonia, diabetes, obesity, fatty liver).
- *Neovascular proliferative diseases,* such as proliferative retinopathy, retinopathy of prematurity (ROP), malignant tumors.
- *Arterial hypertension and atherosclerosis and related diseases* such as coronary heart disease, cardiac arrhythmia, chronic heart failure, nephrosis syndrome, ischemia-reperfusion, peripheral vascular diseases, acute or chronic cerebral ischemia, stroke.
- *Age-related degenerative diseases* such as Alzheimer's, diseases, Parkinson's disease, amyotropic lateral sclerosis, age-related macular degeneration, glaucoma, cataract, otosclerosis, osteoporosis, osteoartritis, sarcopenia, hairlessness (baldness), age-related skin changes.
- *Inflammation caused by soft tissue damage,* such as postoperative inflammation, sport injuries, exhaustive sport activity (marathon running), contusions, burning, frost-bites
- *Inflammation related to neoplasia,* such as prostate and uterus benign or malign tumors, breast cancer, lung cancer, colon cancer, and lymphomas, as well as for treating chemotherapy associated inflammations.
- *Inflammation evoked by common vaccines*, such as influenza (flu), hepatitis, BCG, poliomyelitis, Di-Per-Te (diphtheria-pertussis-tetanus), epidemic parotitis, measles and anti-allergic vaccines.
- *Eye diseases*, such as uveitis, diabetic retinopathy age-related macular degeneration, glaucoma, cataract.

The present invention also relates to the *preparation of the compositions* according to the invention for treating the above-disclosed diseases.

A further embodiment of this invention is the use of compositions as described here for preparing an adjuvant to
- Gene-transfer, natural or semi-synthetic or synthetic DNA o RNA transfers.
- Culture medium either for cell-cultures, or tissue-cultures or bacterial cultures
- support survival of transplanted stem cells and organs.
In these cases reduction of lipid peroxide generation and a metabolic support is achieved.
Our invention is illustrated in details by the following examples, without the intention to restrict our claims to these examples.

### EXAMPLES

### I. BIOLOGICAL EXAMPLES (COMPARATIVE)

Remark: In some of the examples of our priority document we used the term "LTD" instead of the now used "LB" for the applied compound: cell free (killed) extract of Lactobacillus in water. We presumed that LTD (lipoteichoic acid) was the active part of the actually added LB but this is not certain.

### Example I/1: Influences of omega 3 fatty acids, vitamin E and CoQ10 on immunogenic inflammation

The purpose of this experimental model was to measure inhibitor influence of omega 3 fatty acids, vitamin E and CoQ10 separately and in combination, using methods disclosed in literature (J. Surg Res. 2002 Sep;107(1):135-139). For evoking inflammation bacterial endotoxin (LPS) was used and changes in levels of a proinflammatory cytokin, TNF-alpha were measured in a monocyte/macrophage (RAW 264,7) culture. Results are summarized in Table 2. It is clean that both combinations show inhibition of inflammation, the 3 member combination a considerably higher one. Both combinations also show synergy being more effective than the sum of the compound effects. Both combinations were more effective than aspirin.

**Table 2**

| Decrease in proinflammatory cytokine levels in immunogenic inflammation | | | | | |
|---|---|---|---|---|---|
| cytokine | control | CoQ10 | Omega 3 + vitamine E | Omega 3 + vitamin E + CoQ10 (Example II/14) | aspirin |
| TNF-alpha | 0% | -3% | -38% | -58% | -21% |

### Example I/2: Influences of omega 3 fatty acids, vitamin E and CoQ10 on neurogenic inflammation (comparative)

Neurogenic inflammation was evoked in newborn (28 days) Sprague Dawley rats by intraperitoneal administration of capsaicin (50 mg/kg of body mass) as described in the literature (Acta Physiol. Hung. 1987;69(3-4):323-32). Twenty-four hours before capsaicin injection 10 ml intravenous infusions each of CoQ10, or omega 3 + vitamin E, or omega 3 + vitamin E + CoQ10 were applied. Each of these combinations contained 1,0 g omega 3, 10 mg vitamin E and 10 mg CoQ10. Intravenous administration of both combinations containing either 2 or 3 compounds modified significantly both acute and chronic effects of capsaicin, as demonstrated on Table 3. Hypotensive shock and subsequent death were prevented by pre-treatment with omega 3. + vitamin E in 26% and in the omega 3 + vitamin E + CoQ10 in 64%, while in the control group only 22% survived. These findings strongly suggest that these pre-treatments reduced the release of proinflammatory neuropeptides, thus inhibited inflammation. Six weeks after the capsaicin injection, corneal transparency and sensitivity returned to normal in the pre-treated groups, while in the control group reduced transparency and sensitivity were observed. These observations strongly suggest that pre-treatment with the omega 3 + vitamin E and CoQ10 combination either protected sensory nerves from capsaicin toxicity and/or supported the regeneration of damaged nerve fibers. If the omega 3 + vitamin E and CoQ10 pre-treatments were separately applied the sum of these protective influences would be significantly lower than those of the composition containing all three compounds.

**Table 3**

| Decrease in acute and chronic neurogenic inflammations | | | | |
|---|---|---|---|---|
| Neurogenic inflammation | control | CoQ10 | Omega 3 + vitamin E | Omega 3 + vitamin E + CoQ10 |
| | | | | Example II/1 |
| Acute features | 22% | 22% | 26% | 64% |
| Chronic features | 20% | 25% | 45% | 100% |

### Example I/3: Influences of omega 3 fatty acids, vitamin E, CoQ10 and LB on endotoxin induced uveitis in vivo.

Experimental uveitis was evoked by administration of bacterial endotoxin into footpad of rats as described in the literature (J. Neurophysiol. 2005:3815-3825.). Immediately after endotoxin administration, 10 ml intravenous infusion of omega 3 + vitamin E, + CoQ10, or omega 3 + vitamin E + cell-free extract of lactobacillus (LB) or omega 3 + vitamin E + CoQ10 + LB were applied respectively while controls received physiological salt solution. This experiment was repeated and the same compositions were applied 7 days before endotoxin administration. Each of these combinations contained .1,0 g omega 3, 10 mg vitamin E and 10 mg CoQ10 and 0,01 mg LB. Symptoms of uveitis (hyperaemia, corneal oedema, state of the humour aqueous) were evaluated and compared to controls as described in the cited publication. (Table 4). In both experiments inflammatory parameters were 60% lower in average in the treated groups as compared to the control one. These observations further suggest that the omega 3 + vitamin E + CoQ10 combination was more effective in post-treatment, while the omega 3 + vitamin E + LB in the pre-treatment.

**Table 4.**

| Influence of lipid emulsion on endotoxin induced uveitis (EIU) | | | | |
|---|---|---|---|---|
| EIU | control | Omega 3 + vitamin E + CoQ10 (Example II/1) | Omega 3 + Vitamin E + LB (Example II/2) | Omega 3 + vitamin E + CoQ10 + LB (Example II/3) |
| Post-treatment | 0% | 63% | 55% | 58% |
| Pre-treatment | 0% | 51% | 59% | 64% |

### Example I/4: Influences of omega 3 fatty acids, vitamin E, CoQ10 and LB on experimental sepsis

Experimental sepsis of rats was evoked by intraperitoneal administration of bacterial endotoxin as described in the literature (Crit. Car. 2006;10(4):R124). Immediately after endotoxin administration 10 ml intravenous infusion of omega 3 + vitamin E + CoQ10, or omega 3 + vitamin E + cell-free extract of killed lactobacillus (LB) or omega 3 + vitamin E + CoQ10 + LB were applied, while controls received a commercially available lipid emulsion. This experiment was repeated and the same compositions were applied 7 days before endotoxin administration. Each of these combinations contained 1,0 g omega 3, 10 mg vitamin E and 10 mg CoQ10 and 0,01mg LB. While the 72 hour survival was 0% in the control group, it was 75% in average in the treated groups. These observations further suggested that the omega 3 + vitamin E + CoQ10 combination was more effective in post-treatment, while the omega 3 + vitamin E + LB in the pre-treatment. The results are shown in Table 5.

**Table 5**

| Influence of lipid emulsions on experimental sepsis | | | | |
|---|---|---|---|---|
| Survival | control | Omega 3 + vitamin E + CoQ10 | Omega 3+ vitamin E + LB | Omega 3+ vitamin E + CoQ10 + LB |
| | | (Example II/1) | (Example II/2) | (Example II/3) |
| Post-treatment | 0% | 79% | 65% | 68% |
| Pre-treatment | 0% | 72% | 79% | 85% |

### Example I/5: influence of omega 3 fatty acids, Vitamin E, CoQ10 and LB on ischemia-reperfusion in vivo

Using well defined experimental conditions an acute cardiac arrest of dogs was evoked by injecting physio-' logical salt solution into the pericardial sac (pericardial tamponade) for 3 to 5 minutes as earlier described by us (Ann Ophthalmol. 1979 Jun;11(6):909-13.). Immediately after resuscitation intravenous lipid infusion was applied containing either 10 g omega 3 fatty acids + 100 mg vitamin E + 100 mg CoQ10 per .100 ml, or 10 g omega 3 + 100 mg vitamin E + 0,1 mg LB per 100 ml. The final dosage was referred to 1 g omega 3/kg/24 hours. Controls received the same amount of physiological salt solution. From sacrificed animals small samples of the retina, heart muscle and skeletal muscle were studied with light and transmission electron microscopy. Special attention was paid to the plasma membrane alterations, and morphometric methods were applied for quantification of abnormalities as compared to controls. Results are shown in Table 6. These findings show that this treatment significantly decreased tissue damage caused by 3 minutes ischemia-reperfusion. The most prominent effects were found in the neuronal cells (retina) and heart muscle, less evident in skeletal muscle. Similar effects of treatment were found after 5 minutes ischemia-reperfusion, but the magnitude of efficacy was estimated about 15% less as compared to those of 3 minutes experiment. There were no significant differences between these two combinations, at least in this experimental model.

**Table 6**

| Influence of omega 3, vitamin E, CoQ10 and LB on membrane damage in ischemia-reperfusion | | | |
|---|---|---|---|
| Membrane damage | control | omega 3 + E vitamin + CoQ10 (examples II/1) | omega 3 + E vitamin + LB (example II/2) |
| Retinal ganglion cell | 0% | 43% | 45% |
| Heart muscle | 0% | 23% | 19% |
| Skeletal muscle | 0% | 10% | 8% |

**Table 7**

| In vitro growth and differentiation of RPE cells influenced by omega 3 fatty acids, Vitamin E, CoQ10 and LB. | | | | | | | |
|---|---|---|---|---|---|---|---|
| RPE | control | CoQ10 | Omega 3 | LB | omega 3 + E vitamin + CoQ10 (example II/3) | omega 3 + E vitamin + LB (example II/14 | omega 3 + E vitamin + CoQ10+ LB (example II/15) |
| growth | 0% | 1% | 23% | 7% | 43% | 69% | 72% |
| differentiation | 0% | 3% | 21 | 4% | 38% | 71% | 67% |

### Example I/6: Influences of omega 3 fatty acids, Vitamin E, CoQ10 and LB on retinal cell-culture

The aim of this study was to reveal the influence of omega 3 fatty acids, Vitamin E, CoQ10 and LB separately or in combination on the development and differentiation of retinal cells in culture (in vitro). Retinas of newborn (1-2 days) Wistar rats were isolated and cultured as described in our previous paper (Int.J.Dev.Neurosc. 1998 Aug;16(5):423-32). In six different combinations, the culture medium contained 10g omega 3 fatty acids, 100 mg vitamin E, 100mg CoQ10 and 0,05 microgram LB per 100 ml. At the 7 day stage size and differentiation of retinal pigment epithelial (RPE) cells were determined by morphometric analysis, as described in the cited work. Our results are presented in Table 7. These in vitro data show that (1) omega 3 + vitamin E + CoQ10 enhanced both growth and differentiation of RPE cells; (2) this effect was higher than the sum of the effects of each of these compounds; (3) addition of LB further improved both cell-growth and differentiation; (4) the same results were obtained without CoQ10 in the culture medium, suggesting probably an enhanced endogen biosynthesis of CoQ10 by LB.

### Example I/7: Influences of cell-free extract of LB omega 3 fatty acids and vitamin E on endotoxin induced uveitis in vivo.

Experimental uveitis was evoked by administration of bacterial endotoxin into footpad of rats as described in the literature (J. Neurophysiol. 2005:3815-3825.). Immediately after endotoxin administration, 10 ml intravenous infusion of omega 3 + vitamin E, or cell-free extract of LB + omega 3 + vitamin E were applied respectively while controls received physiological salt solution. This experiment was repeated and the same compositions were applied 7 days before endotoxin administration. Each of these combinations contained 1,0 g omega 3, 10 mg vitamin E and 10 mg LB. Symptoms of uveitis (hyperaemia, corneal oedema, state of the humour aqueous) were evaluated and compared to controls as described in the cited publication. (Table 4). In both experiments inflammatory parameters were 60% lower in average in the treated groups as compared to the control one. These observations further suggest that the LB was more effective in post-treatment, while the LB + omega 3 + vitamin E in the pre-treatment. A omega 3 + vitamin E showed weak effect in both pre and post-treatment (* not formulated for Exsamples)

**Table 8**

| EIU | control | LB (Example II/1) | Omega 3 + vitamin E* | LB + Omega 3 + vitamin E (Example II/2) |
|---|---|---|---|---|
| Post-treatment | 0% | 63% | 25% | 58% |
| Pre-treatment | 0% | 59% | 11% | 64% |

### Example I/8: Influence of LB, omega 3 fatty acids and Vitamin E on ischemia-reperfusion in vivo

Using well defined experimental conditions an acute cardiac arrest of dogs was evoked by injecting physiological salt solution into the pericardial sac (pericardial tamponade) for 3 to 5 minutes as earlier described by us (Ann Ophthalmol. 1979;11:909-13.). Immediately after resuscitation intravenous lipid infusion was applied containing either 10mg LB or 10g omega 3 fatty acids + 100mg vitamin E per 100 ml, or 10mg LB + 10g omega 3 + 100mg vitamin E per 100 ml. The final dosage was referred to 1 g omega 3/kg/24 hours. Controls received the same amount of physiological salt solution. From sacrificed animals small samples of the retina, heart muscle and skeletal muscle were studied with light and transmission electron microscopy. Special attention was paid to the plasma membrane alterations, and morphometric methods were applied for quantification of abnormalities as compared to controls. Results are shown in Table 6. These findings show that this treatment significantly decreased tissue damage caused by 3 minutes ischemia-reperfusion. The most prominent effects were found in the neuronal cells (retina) and heart muscle, less evident in skeletal muscle. Similar effects of treatment were found after 5 minutes ischemia-reperfusion, but the magnitude of efficacy was estimated about 15% less as compared to those of 3 minutes experiment. There were no significant differences between these two combinations, at least in this experimental model. Neither LB alone nor a combination of omega 3 + vitamin E showed weak effect in both pre and post-treatment (* not formulated for Examples)

**Table 9**

| Membrane damage | control | LB (Example II/1) | omega 3 + E vitamin (*) | LB + omega 3 + E vitamin (example II/2) |
|---|---|---|---|---|
| Retinal ganglion cell | 0% | 10% | 13% | 45% |
| Heart muscle | 0% | 8% | 15% | 19% |
| Skeletal muscle | 0% | 7% | 10% | 18% |

### Example I/9: Influence of LB, omega 3 fatty acids and Vitamin E on metabolic syndrome

Metabolic is a cluster of diseases characterized by(i) Impaired lipid metabolism (high total LDL cholesterol and trigliceride levels , and low HDL levels in the plasma) ii) Impaired glucose metabolism (positive glucose, tolerance test or definitively high glucose levels in the plasma, and, an increased insulin levels in the plasma). Clinical manifestation of the metabolic syndrome are (i) Arterial hypertension, (ii) Type 2 (non-insulin dependent) diabetes, (iii) Obesity and (iv) Fatty liver. Recent studies suggested that chronic low-grade bacterial infections play a central role in the pathogenesis of these diseases. They may occur alone but more frequently they combine each with other. We selected 60 patients with age 50-60 each them affected by diabetes and high blood lipid levels. They were randomly selected into two groups, 30 patient in each. In addition to their current treatment, which remained unchanged during the study period, the following additional treatments were applied: The control group received a multivitamin every day, while the *treated* group received full extract of LB in intramuscular injection (0,32 mg), ones every 14 days and, a commercially available soft-gel composition of omega 3 FA (1000mg) + vitamin E (10mg) every day. This treatment was followed for 3 months. Labor tests and visual field test were performed at the beginning and at the end of this study. Results are summarized in the Table 10

**Table 10**

| | Control (Multivitamin) | LB + omega 3 + vitamin E (Example II/3 | p |
|---|---|---|---|
| Total cholesterol | +8% | -5% | <0.01 |
| HDL cholesterol | -4% | +9% | <0.05 |
| Triglyceride | 0% | -8% | ns |
| Glucose | +3 | -5% | ns |
| CRP | +2% | -8% | <0.01 |
| Blood pressure | +3% | -5% | ns |
| Visual Field Defect | 0% | -10% | <0.05 |

Results: both laboratory tests and visual field test improved in the treated group while worsened or unchanged in the control group. These changes were not significant compared to the baseline, but the improvement of total cholesterol, HDL cholesterol and CRP levels and visual field were statistically significant if were compared to the controls. These findings clearly demonstrated an excellent therapeutic efficacy of this combination of killed LB and omega 3 FA and vitamin E. These results are further supported by the facts that the treatment period was very short for a chronic disease like the metabolic syndrome.
(*) For the above experiments the following commercially available compounds were used:
- omega 3: lipid emulsion containing fish oil LB : cell free extract of killed Lactobacillus in water (Gynevac®, Vakcina Ltd, Sajogalgóc, Hungary
- capsaicin: Sigma-Aldrich, St. Louis, MO,
- Endotoxin: Escherichia coli LPS (Sigma-Aldrich, St. Louis, MO., USA))
- control: lipid emulsion: soy oil-containing aqueous emulsion.

### II. EXAMPLE SFOR COMPOSITION

### Example II/1: Injection for i.v use

- full extract of killed Lactobacillus 10mg
- water for injection 1,0ml

### Example II/2: injection for i.v. use

- full extract of killed Lactobacillus 0,5mg
- omega 3 fatty acids 500,0 mg
- α-tocopherol 10 mg
- water for injection q.s. ad 10 ml
These compounds are mixed using the habitual technology for preparing a lipid emulsion, using the following excipients: glycerol, egg lecithin, and sodium hydroxide for pH adjustment.

### Example II/3: Infusion

### Composition

- cytoplasmic extract of Bifidobacterium 1 mg
- EPA 5,0 g
- DNA 2,5 g
- α-tocopherol 0,1 g
- water for injection q.s. ad 100 ml
These compounds are mixed using the habitual technology for preparing a lipid emulsion, using the following excipients: glycerol, soy lecithin, and sodium hydroxide for ph adjustment

### Example II/4a Soft gel

### Composition

- cytoplasmic extract of Lactobacillus 50 mg
- EPA 500 mg
- DHA 250 mg
- α-tocopherol 10 mg
- water for injection q.s.
These compounds are mixed using the habitual technology for preparing a lipid emulsion in soft gel, using the following excipients: glycerol, egg lecithin, and sodium hydroxide for pH adjustment.

### Example II/4b Infusion

### Composition

- cytoplasmic extract of Lactobacillus 1,0 mg
- cytoplasmic extract of Bifidobacterium 1,0 mg
- EPA 500 mg
- DHA 250 mg
- α-tocopherol 10 mg
- vitamin B1 10 mg
- water for injection q.s. ad 100 ml
These compounds are mixed using the habitual technology for preparing a lipid emulsion, using the following excipients: glycerol,e lecithin, and sodium hydroxide for pH adjustment

### Example II/5: soft gel

### Composition

- full extract of killed Saccharomyces cerevisie 50mg
- omega 3 fatty acids 500mg
- α-tocopherol 20mg
- vitamin A 0.000 IU
- water for injection q.s.
These compounds are mixed using the habitual technology for preparing a lipid emulsion in soft gel using the following excipients: glycerol, egg lecithin, and sodium hydroxide for pH adjustment

### Example II/6: Infusion

### Composition

- full extract of killed Lactobacillus 1,0mg
- DHA 1000mg
- CoQ10 10mg
- α-tocopherol 10mg
- alpha lipoic acid 10mg
- water for injection q.s. ad 100 ml
These compounds are mixed using the habitual technology for preparing a lipid emulsion, using the following excipients: glycerol, egg lecithin, and sodium hydroxide for pH adjustment

### Example II/7: Solution

### composition

- full extract of killed Lactobacillus 1mg
- EPA 50 mg
- DHA 25 mg
- α-tocopherol 1mg
- selected DNA/RNA 1mg
- water for injection q.s. ad 1 ml
The compounds are mixed using the habitual technology for preparing a lipid emulsion, using the following excipients: glycerol, egg lecithin, and sodium hydroxide for ph adjustment

### Example II/8: Injection

### Composition

- full extract of killed Lactobacillus 10 mg
- full extract of killed Bifidobacterium 10 mg
- EPA 50 mg
- DHA 25 mg
- α-tocopherol 1mg
- water for injection q.s. ad 1 ml
These compounds are mixed using the habitual technology for preparing a lipid emulsion, using the following excipients: glycerol, egg lecithin, and sodium hydroxide for pH adjustment

### Example II/9: Injection

### Composition

- full extract of Lactobacillus 1mg
- full extract of Bifidobacterium 1mg
- DHA etylester 200mg
- EPA etylester 100mg
- α-tocopherol hydrosoluble 10mg
- water for injection q.s. ad , 10 ml
These compounds are mixed using the habitual technology for preparing a lipid emulsion, using the following excipients, glycerol, egg lecithin, and sodium hydroxide for pH adjustment

### (Example II/10: Qintment/gel

### Composition

- full extract of killed Lactobacillus 1 mg
- full extract of killed Bifidobacterium 1 mg
- DHA 200 mg
- EPA 20 mg
- α-tocopherol 10mg
- water for injection q.s. ad 10 g
The compounds are mixed using the habitual technology for preparing a lipid emulsion, using the following excipients: water for injection q.s. ad 100 g hydrophilic ointment/gel, glycerol, soy lecithin, and sodium hydroxide for pH adjustment

### Example II/11: Gel

### Composition:

- full extract of killed Lactobacillus 10 mg
- DHA 9,0 g
- EPA 1,0 g
- α-tocopherol 0,1 g
- CoQ10 0,1 g
- Gel-forming vehicle* and 100,0 g
These compounds are mixed using the habitual technology for preparing a gel. (*e.g. USP 5,268,112)

### Example II/12: Soft-gel capsule

### Composition:

- full extract of killed Lactobacillus 10 mg
- full extract of killed Bifidobacteria 10mg
- DHA 750 mg
- EPA 50 mg
- α-tocopherol 10 mg
These compounds are mixed using the habitual technology for preparing lipid emulsion, using the following excipients: glycerol, soy lecithin, water for injection q.s., and vegetal gelatine for capsules.

### Example II/ 13: Solution/eye drop

### Composition:

- full extract of killed Lactobacillus 1mg
- DHA-ethyl-ester 90mg
- EPA-ethyl-ester 10mg
- α-tocopherol ethyl-ester 1mg
- water for injection q.s. ad 10 ml
The compounds are mixed using the habitual technology for preparing a lipid emulsion, using the following excipients: Glycerol, polyvinyl alcohol, and Sodium hydroxide for pH adjustment.

### Example II/15: Medium for cell or tissue culture

### Composition

- full extract of killed Lactobacillus 0,5 mg
- full extract of killed Bifidobacterium 0,5 mg
- DHA 250 mg
- EPA 50 mg
- α-tocopherol 10 mg
- Dulbecco medium ad 100 ml
The compounds are mixed using the habitual technology for preparing a lipid emulsion, using The following excipients: glycerol, soy lecithin, and sodium hydroxide for pH adjustment.

## Claims

1. A stable lipid emulsion for the manufacture of a medicament for preventing, treating and attenuating inflammatory diseases caused by elementary inflammation in the Plasma Membrane Redox System (PMRS) of cells involved in inflammation by way of inhibiting of harmful lipid peroxide generation,
said emulsion comprising
a) as biologically active ingredient a combination consisting of:
i) at least one killed probiotic and
ii) at least one omega-3 fatty acid and
iii) vitamin E and
b) as formulation additives water and a pharmaceutically acceptable emulgator where the particle size in the lipid emulsion is 0,001 to 100 microns, and
where the mass ratio of omega-3 fatty acid to water is 100: 1 to 10000.

2. A stable lipid emulsion according to claim 1 comprising as the emulgator lecithin, bile, bile acids, Tween® MT or polyvinyl alcohol.

3. A stable lipid emulsion according to any of claims 1 to 2 for the manufacture of a medicament for preventing, treating and attenuating inflammatory diseases caused by elementary inflammation in the Plasma Membrane Redox System (PMRS) of cells involved in inflammation by way of inhibiting of harmful lipid peroxide generation,
said emulsion comprising
a) at least one killed probiotic and
b) at least one omega-3 fatty acid and
c) vitamin E and
d) ubiquinone.

4. A stable lipid emulsion according to any one of claims 1 to 3 comprising as biologically active ingredients the full extract of at least one killed probiotic where said extract is a member of the group consisting of the cytoplasmic fraction of killed probiotics, the nucleotide components (DNA, RNA) of probiotics and combinations thereof.

5. A stable lipid emulsion according to any one of claims 1 to 4 where the probiotic is selected from the group consisting of Lactobacillus acidophilus, Lactobacillus casei, Lactobacillus plantarum, Lactobacillus reuteri, Lactobacillus rhamnosus, Lactobacillus GG, Lactobacillus bulgaricus, Lactobacillus bifidus, Lactobacillus caucasicus, Lactobacillus brevis, Lactobacillus cellobiosus, Lactobacillus crispatus, Lactobacillus curvatus, Lactobacillus fermentum, Lactobacillus gasseri, Lactobacillus johnsonii. Lactobacillus salivarus, Bifidobacterium animalis subsp. lactis, Bifidobacterium bifidum, Bifidobacterium breve, Bifidobacterium infantis, Bifidobacterium longum, Bifidobacterium adolescentis, Bifidobacterium animalis, Bifidobacterium thermophilum, Bifidobacterium lactis, Lactococcus lactis (formerly known as Streptococcus lactis), Streptococcus thermophilus, Bacillus coagulans, Enterococcus faecalis, Enterococcus faecium, Saccharomyces boulardii, Saccharomyces cerevisiae, Monascus purpurea.

6. A stable lipid emulsion according to any one of claims 1 to 5 where the omega-3 fatty acid is a pharmacologically identical natural form or source of alpha-linoleic acid, and eicosapentaenoic acid and docosahexaenoic acid and esters thereof, namely a member of the group consisting of the ethyl-ester and triglyceride, phospholipids, preferably phosphatidyl-inositol, phosphatidyl-choline, phosphatidyl-ethanolamine, phosphatidyl-serine and sphingomyelin, and combinations thereof; where the vitamin E is a member of the group consisting of pure α-, β-, γ-, and δ-tocopherol, α-, β-, γ-, and δ-tocotrienol and their natural, semi-synthetic and synthetic esters, and where omega-3 fatty acid is in the form of one of its precursors selected from the group consisting of fish-oil, plant-oil, a combination thereof, a fish-oil derivate from any part of a see-fish selected from a member of the group consisting of salmon, cod-liver, other marine organisms and vegetable oil selected from the group consisting of linseed-oil, rape oil, grape pip oil and micro-algae oil and their combination.

7. Composition in an aqueous solution for elementary inhibition of inflammation in human and mammal organism by inhibition of harmful lipid peroxide generation in the Plasma Membrane Redox System (PMRS) of cells involved in inflammation comprising
a) as biologically active ingredient a combination for elementary inhibition of inflammation consisting of:
i) at least one killed probiotic and
ii) water-soluble salt or ester of at least one omega-3 fatty acid and
iii) water-soluble ester of natural semi-synthetic or synthetic vitamin E, and
b) as pharmaceutically acceptable formulation additives water and water-soluble formulation additives, excipients, vehicles, preservatives and colorants
and **characterized by** the following mass ratios of ingredients:
i) Omega-3 fatty acids : vitamin E = 100 : 1 to 100;
ii) Omega-3 fatty acids : probiotics = 100 : 0,01 to 100.

8. .
A composition according to any of claims 1 to 4 further comprising pharmacologically compatible excipients selected of the group consisting of excipients for parenteral (intravenous, intramuscular, intradermal, intraarticular, intraocular, intralesional, subcutaneous) application, for enteral (oral, transrectal) or nasal application, for direct topical medication selected of the group eye-drops, gel, spray, ointment, lotions, for liposome encapsulated delivery, and for soft-gel.

9. Composition according of any of claims 1 to 8 for the manufacture of a medicament which can be administered parenterally, in endovenous infusion, in an injection, subcutaneous injection, intramuscular injection, or for intradermal use, intra-lesional use, para-lesional use, intraarticular use, intraocular use, or administered enterally, orally, transrectally and topically in eye-drops, gel, spray, ointment, lotions and in liposomes.

10. A composition according to any of claims 1 to 8 for the manufacture of a medicament for preventing, treating, attenuating inflammations
a) related to bacterial, viral and fungal infections;
b) evoked by common vaccines selected of the group consisting of influenza (flu), hepatitis, BCG, poliomyelitis, Di-Fer-Te (diphtheria-pertussis-tetanus), epidemic parotitis, measles and antiallergic vaccines,
c) of mucous membranes selected of members of the group consisting of conjunctivitis, periodotitis, oesophagitis, reflux disease, gastritis, enteritis, colitis, cholecystitis, cystitis, pyelonephritis, sinusitis, bronchitis, vaginitis, prostatitis, and related diseases of the group consisting of hepatitis, cirrhosis, nephritis, pleuritis and cystic fibrosis,
d) rheumatoid arthritis, juvenile (type I) diabetes, Crohn's disease, colitis ulcerosa, psoriasis, lupus erythematous and multiplex sclerosis,
e) neuropsychiatric diseases, selected from the group consisting of schizophrenia, depression, anxiety and panic-disease,
f) allergenic inflammation selected from the group consisting of bronchial asthma, atopic dermatitis, hay―fever, allergic conjunctivitis, allergic rhinitis,
g) the metabolic syndrome (dyslipidemia, arterial hypertension, type 2 diabetes, obesity, fatty liver)
h) proliferative neovascular diseases selected from the group consisting of proliferative retinopathy, retinopathy of prematurity (ROP) and malignant tumors,
i) age-related degenerative diseases selected from of the group consisting of Alzheimer's disease, Parkinson's disease, amyotropic lateral sclerosis, otosclerosis, osteoporosis, osteoarthritis, sarcopenia, hairlessness and age-related skin changes,
j) soft tissue damage caused by postoperative inflammation, sport injures, extreme sport activities, contusions, burnings, frostbites,
k) neoplasia, prostate and uterus benign and malign tumors, breast cancer, lung cancer, colon cancer, and lymphomas, as well as for treating chemotherapy associated inflammations,
l) eye diseases, uveitis, diabetic retinopathy, age-related macular degeneration, glaucoma, cataract,
m) arterial hypertension and atherosclerosis and related diseases selected from the group consisting of coronary heart disease, cardiac arrhythmia, chronic heart failure, nephrosis syndrome, ischemia-reperfusion, peripheral vascular diseases, acute cerebral ischemia, chronic cerebral ischemia and stroke.

11. Composition according to any of claims 1 to 8 for the manufacture of a medicament for preventing the following diseases caused by elementary inflammation: arterial hypertension and atherosclerosis and related diseases, coronary heart disease, cardiac arrhythmia, chronic heart failure, nephrosis syndrome, ischemia-reperfusion, peripheral vascular diseases, acute cerebral ischemia, chronic cerebral ischemia and stroke.

12. Composition according to any of claims 1 to 8 for the manufacture of a medicament for preventing, treating, attenuating ischemia, ischemia-reperfusion, acute and chronic cerebral ischemia.

13. Use of compositions according to any of claims 1 to 8
a) for preparing a metabolic adjuvant to gene-transfer of natural, semi-synthetic and synthetic DNA and RNA, and
b) for preparing a metabolic adjuvant to a culture medium for cell-culture, tissue-culture and bacterial-culture, and
c) for preparing a metabolic adjuvant to support survival of transplanted stem cells and organs.

14. Use of compositions according to any of claims 1 to 8 for preparing omega-3 fatty acid and vitamin E enriched cell and tissue extracts, food supplements, foods and nutrients for human and veterinary application.

15. A composition according to any of claims 1 to 8 further containing at least one pharmacologically compatible biologically active substance selected from the group consisting of vitamin A, B, C, D, F, K, sex-steroids, metal ions of the group sodium, calcium, magnesium, potassium, zinc, amino-carnitine, alpha-lipoic acid, glutathion, essential amino acids, the alkaloid berberine, glycosamino-glycans of the group hyaluronic acid, chondroitin-sulphate, heparin, heparin-sulphate.

## Patentansprüche

1. Eine stabile Lipidemulsion für die Herstellung eines Medikaments zur Vorbeugung, Behandlung und Linderung von Entzündungskrankheiten verursacht durch elementare Entzündung im retikulären Plasmamembransystem (PMRS) der entzündeten Zellen mittels Unterbinden der Bildung schädlicher Lipidperoxide,
die genannte Emulsion enthaltend
a) als biologisch aktiven Wirkstoff ein Gemisch bestehend aus
i) wenigstens einem abgetöteten Probiotikum und
ii) wenigstens einer Omega-3-Fettsäure und
iii) Vitamin E und
b) als Formulierungszutaten Wasser und einen pharmazeutisch akzeptablen Emulgator,
wobei die Teilchengröße in der Lipidemulsion 0,001 bis 100 Mikrometer und
das Massenverhältnis von Omega-3-Fettsäure zu Wasser 100 : 1 bis 10000 beträgt.

2. Eine stabile Lipidemulsion gemäß Anspruch 1 enthaltend als Emulgator Lezithin, Gallenflüssigkeit, Gallensäuren, Tween® MT oder Polyvinylalkohol.

3. Eine stabile Lipidemulsion gemäß einem beliebigen der Ansprüche 1 oder 2 für die Herstellung eines Medikaments zur Vorbeugung, Behandlung und Linderung von Entzündungskrankheiten verursacht durch elementare Entzündung im retikulären Plasmamembransystem (PMRS) mittels Unterbinden der Bildung schädlicher Lipidperoxide,
die genannte Emulsion enthaltend
a) wenigstens ein abgetötetes Probiotikum und
b) wenigstens eine Omega-3-Fettsäure und
c) Vitamin E und
d) Ubichinon.

4. Eine stabile Lipidemulsion gemäß einem beliebigen der Ansprüche 1 bis 3 enthaltend als biologisch aktive Wirkstoffe den ganzen Extrakt wenigstens eines abgetöteten Probiotikums, wobei der Extrakt unter Zytoplasmafraktionen abgetöteter Probiotika, nukleotiden Komponenten (DNA, RNA) von Probiotika und deren Kombinationen ausgewählt wird.

5. Eine stabile Lipidemulsion gemäß einem beliebigen der Ansprüche 1 bis 4, wobei das Probiotikum unter Lactobacillus acidophilus, Lactobacillus casei, Lactobacillus plantarum, Lactobacillus reuteri, Lactobacillus rhamnosus, Lactobacillus GG, Lactobacillus bulgaricus, Lactobacillus bifidus, Lactobacillus caucasicus, Lactobacillus brevis, Lactobacillus cellobiosus, Lactobacillus crispatus, Lactobacillus curvatus, Lactobacillus fermentum, Lactobacillus gasseri, Lactobacillus johnsonii. Lactobacillus salivarus, Bifidobacterium animalis subsp. lactis, Bifidobacterium bifidum, Bifidobacterium breve, Bifidobacterium infantis, Bifidobacterium longum, Bifidobacterium adolescentis, Bifidobacterium animalis, Bifidobacterium thermophilum, Bifidobacterium lactis, Lactococcus lactis (früher als Streptococcus lactis bekannt), Streptococcus thermophilus, Bacillus coagulans, Enterococcus faecalis, Enterococcus faecium, Saccharomyces boulardii, Saccharomyces cerevisiae und Monascus purpurea ausgewählt wird.

6. Eine stabile Lipidemulsion gemäß einem beliebigen der Ansprüche 1 bis 4, wobei die Omega-3-Fettsäure eine pharmazeutisch identische, natürliche Form oder Quelle von Alpha-Linolensäure und Eicosapentaensäure und Docosahexaensäure und deren Estern ist, nämlich ausgewählt unter Ethylester und Triglizerid, Phodpholipiden, vorteilhaft Phosphatidyl-inositol, Phosphatidyl-cholin, Phosphatidyl-ethanolamin, Phosphatidyl-serin und Sphingomyelin und deren Kombinationen; wobei das Vitamin E unter reinem α-, ß-, γ- und δ-Tocopherol, α-, ß-, γ- und 8-Tocotrienol und deren natürlichen, halbsynthetischen und synthetischen Estern ausgewählt wird und die Omega-3-Fettsäure in einer der Prekursor-Formen vorliegt, ausgewählt unter Fischöl, Pflanzenöl, deren Kombination, Fischölderivat, letzteres gewonnen aus beliebigen Teilen von Seefischen, ausgewählt unter Lachs, Dorschleber und anderen Seelebewesen, sowie Pflanzenöl, ausgewählt unter Leinsamenöl, Rapsöl, Traubenkernöl, Mikroalgenöl und deren Kombinationen.

7. Arzneimittelzusammensetzung in einer wässerigen Lösung zur elementaren Verhinderung von Entzündungen in menschlichen und Säugetierorganismen durch Unterbinden der Bildung schädlicher Lipidperoxide im retikulären Plasmamembransystem (PMRS) der entzündeten Zellen, enthaltend
a) als biologischen Wirkstoff zur elementaren Verhinderung der Entzündung ein Gemisch, bestehend aus
i) wenigstens einem abgetöteten Probiotikum und
ii) wenigstens einem wasserlöslichen Salz oder Ester wenigstens einer Omega-3-Fettsäure und
iii) einem wasserlöslichen Ester von natürlichem, halbsynthetischem oder synthetischem Vitamin E, und
b) als pharmazeutisch akzeptable Formulierungsadditive Wasser und wasserlösliche Formulierungsadditive, Bindemittel, Träger, Konservierungsmittel und Färbemittel,
und charakterisiert durch folgende Massenverhältnisse der Wirkstoffe:
i) Omega-3-Fettsäuren : Vitamin E = 100 : 1 bis 100;
ii) Omega-3-Fettsäuren : Probiotika = 100 : 0,01 bis 100.

8. Zusammensetzung gemäß einem beliebigen der Ansprüche 1 bis 4 enthaltend ferner pharmazeutisch kompatible Bindemittel, ausgewählt unter Bindemitteln für parenterale (intravenöse, intramuskuläre, intradermale, intraarterielle, intraokulare, intralesionale, subkutane) Anwendung, enterale (orale, transrektale) Anwendung, direkte örtliche Verabreichung von Medikamenten, ausgewählt unter Augentropfen, Gel, Spray, Salben, Lotion, sowie für Liposomkapsel-Verabreichung und für Softgel.

9. Zusammensetzung gemäß einem beliebigen der Ansprüche 1 bis 8 für die Herstellung eines Medikaments, welches parenteral mittels intravenaler Infusion, Injektion, subkutaner Injektion, intramuskularer Injektion verabreicht, oder intradermal, intralesional, paralesional, intraartiriell und intraokular angewandt oder enteral, oral, transrektal und örtlich in Augentropfen, Gels, Sprays, Salben, Lotionen und Liposomen verabreicht werden kann.

10. Zusammensetzung gemäß einem beliebigen der Ansprüche 1 bis 8 für die Herstellung eines Medikaments zur Vorbeugung, Behandlung und Linderung von
a) Entzündungen im Zusammenhang mit bakteriellen, viralen und Pilzinfektionen,
b) Entzündungen hervorgerufen durch gebräuchliche Impfstoffe, ausgewählt unter Impfstoffen gegen Grippe, Hepatitis, BCG, Poliomyelitis, Di-Per-Te (Diphtheria-Pertussis-Tetanus), epidemische Parotitis, Masern und antiallergischen Impfstoffen,
c) Entzündungen der Schleimhaut, ausgewählt unter Konjunktivitis, Periodotitis, Oesophagitis, Refluxkrankheit, Gastritis, Enteritis, Colitis, Cholecystitis, Cystitis, Pyelonephritis, Sinusitis, Bronchitis, Vaginitis, Prostatitis und damit verbundenen Krankheiten, ausgewählt unter Hepatitis, Zirrhose, Nephritis, Pleuritis und cystische Fibrose,
d) rheumatoide Arthritis, Juvenile Diabetes (Typ I), Crohn-Krankheit, Colitis ulcerosa, Schuppenflechte, Lupus erythematous, Multiple Sklerose,
e) neuropsychiatrischen Krankheiten, ausgewählt unter Schizophrenie, Depression, Dysphorie und Panikattacke,
f) allergische Entzündungen, ausgewählt unter Asthma bronchiale, atopische Dermatitis, Heuschnupfen, allergische Konjunktivitis und allergische Rhinitis,
g) metabolischen Syndromen (Dyslipidemie, arterielle Hypertonie, Diabetes Typ 2, Fettsucht, Fettleber),
h) proliferativen neovaskularen Krankheiten, ausgewählt unter proliferativer Retinopathie, Frühgeboren-Retinopathie (ROP) und bösartigen Tumoren,
i) altersbedingten degenerativen Krankheiten, ausgewählt unter Alzheimer-Krankheit, Parkinson-Krankheit, amyotropischer lateraler Sklerose, altersbedingter makularer Degeneration, Grünem Star, Grauem Star, Otosklerose, Osteoporose, Osteoarthritis, Sarkopenie, Haarlosigkeit und altersbedingten Änderungen der Haut,
j) Beschädigungen weichen Körpergewebes verursacht durch postoperative Entzündungen, Sportverletzungen, Extremsport-Betätigungen, Prellungen, Verbrennungen, Erfrierungen,
k) Neoplasie, benignen und malignen Prostata- und Uterustumoren, Brustkrebs, Lungenkrebs, Darmkrebs, Lymphom, sowie Behandlung von Entzündungen in Verbindung mit Chemotherapie,
l) Augenkrankheiten, Uveitis, diabetische Retinopathie, altersbedingter makularer Degeneration, Grauem Star, Grünem Star,
m) arterieller Hypertonie und Atherosklerose sowie damit verbundenen Krankheiten, ausgewählt unter koronaren Herzkrankheiten, Herzarrhythmie, chronischem Herzversagen, Nephrosis-Syndrom, Ischämie-Reperfusion, Krankheiten der peripheren Gefäße, akuter zerebraler Ischämie, chronischer zerebraler Ischämie, Schlaganfall.

11. Zusammensetzung gemäß einem beliebigen der Ansprüche 1 bis 8 für die Herstellung eines Medikaments zur Vorbeugung folgender, durch elementare Entzündung verursachte Krankheiten: arterielle Hypertonie und Atherosklerose und damit verbundene Krankheiten, koronare Herzkrankheiten, Herzarrhythmie, chronisches Herzversagen, Nephrosis-Syndrom, Ischämie-Reperfusion, Krankheiten der peripheren Gefäße, akute zerebrale Ischämie, chronische zerebrale Ischämie und Schlaganfall.

12. Zusammensetzung gemäß einem beliebigen der Ansprüche 1 bis 8 für die Herstellung eines Medikaments zur Vorbeugung, Behandlung und Linderung von Ischämie, Ischämie-Reper-fusion, akuter und chronischer zerebraler Ischämie.

13. Anwendung von Zusammensetzungen gemäß einem beliebigen der Ansprüche 1 bis 8
a) für die Herstellung eines metabolischen Adjuvans zum Gentransport natürlicher, halbsynthetischer oder synthetischer DNA und RNA, und
b) für die Herstellung eines metabolischen Adjuvans zur Zubereitung von Nährmedien für Zell-, Gewebe- und Bakterienkulturen, und
c) für die Herstellung eines metabolischen Adjuvans zur Förderung des Überlebens von transplantierten Stammzellen und Organen.

14. Anwendung von Zusammensetzungen gemäß einem beliebigen der Ansprüche 1 bis 8 für die Herstellung von mit Omega-3-Fettsäure und Vitamin E angereicherten Zell- und Gewebeextrakten, Lebensmittelzusätzen, Speisen und Nährstoffen zur humanen und veterinären Anwendung.

15. Zusammensetzung gemäß einem beliebigen der Ansprüche 1 bis 8 enthaltend wenigstens eine pharmakologisch kompatible, biologisch aktive Substanz, ausgewählt unter Vitaminen A, B, C, D, F und K, Sexsteroiden, Metallionen, ausgewählt unter Natrium, Kalzium, Magnesium, Kalium und Zink, Carnitin, Alpha-Fettsäuren, Glutathionen, wichtigen Aminosäuren, das Alkaloid Berberin und Glycosaminoglykane, ausgewählt unter Hyaluronsäure, Chondroitinsulfat, Heparin und Heparinsulfat.

## Revendications

1. Une émulsion lipidique stable pour la production d'un médicament pour prévenir, traiter et atténuation causée par l'inflammation élémentaire dans la Membrane Plasmique Redox Système (PMRS) des cellules impliquées dans l'inflammation au moyen d'inhibition de la génération des peroxydes lipidiques pernicieux ladite émulsion comprenant
a) comme ingrédient biologiquement actif une combinaison composée de :
i) au moins un probiotique tué et
ii) au moins un acide gras oméga-3 et
iii) vitamine E et
b) comme additifs de formulation de l'eau et un emulgator pharmaceutiquement acceptable
où la mésure des particules dans l'émulsion lipidique est de 0,001 à 100 microns, et
où le rapport de masse de l'eau à l'acide gras oméga-3 est de 100:1 à 10000.

2. Une émulsion lipidique stable selon la revendication 1 comprenant comme emulgator la lécithine, la bile, des acides biliaires, Tween ® MT ou l'alcool polyvinylique.

3. Une émulsion lipidique stable selon quelquonque des revendications 1 à 2 pour la production d'un médicament pour prévenir, traiter et atténuation causée par l'inflammation élémentaire dans la Système Membrane Plasmique Redox (PMRS) des cellules impliquées dans l'inflammation au moyen d'inhibition de la génération peroxydes lipidiques pernicieux, ladite émulsion comprenant
i) au moins un probiotique tué et
ii) au moins un acide gras oméga-3 et
iii) vitamine E et
iv) ubiquinone.

4. Une émulsion stable de lipides selon quelquonque des revendications 1 à 3 comprenant comme ingrédients biologiquement actifs l'extrait complet de au moin un probiotique tué où ledit extrait est membre du la groupe composée de_la fraction cytoplasmique des probiotiques tués, les composants de nucléotides (ADN, ARN) de probiotiques et de leurs combinaisons.

5. Une émulsion stable de lipides selon quelquonque des revendications 1 à 4 où le probiotique est choisi de la group composée de Lactobacillus acidophilus, Lactobacillus casei, Lactobacillus plantarum, Lactobacillus reuteri, Lactobacillus rhamnosus, Lactobacillus GG, Lactobacillus bulgaricus, Lactobacillus bifidus, Lactobacillus caucasicus, Lactobacillus brevis, Lactobacillus cellobiosus, Lactobacillus crispatus, Lactobacillus curvatus, Lactobacillus fermentum, Lactobacillus gasseri, Lactobacillus johnsonii. Lactobacillus salivarus, Bifidobacterium animalis subsp. lactis, Bifidobacterium bifidum, Bifidobacterium breve, Bifidobacterium infantis, Bifidobacterium longum, Bifidobacterium adolescentis, Bifidobacterium animalis, Bifidobacterium thermophilum, Bifidobacterium lactis, Lactococcus lactis (auparavant Streptococcus lactis), Streptococcus thermophilus, Bacillus coagulans, Enterococcus faecalis, Enterococcus faecium, Saccharomyces boulardii, Saccharomyces cerevisiae, Monascus purpurea.

6. Une émulsion stable de lipides selon quelquonque des revendications 1 à 5, où l'acide gras oméga-3 est une forme naturelle identique sur le plan pharmacologique ou la source de l'acide alpha linoleic et l'acide eicosapentaénoïque et l'acide docosahexaénoïque et leurs esters, à savoir un membre du groupe composé de l'ester d'éthyle et des triglycérides, des phospholipides, de préférence phosphatidyl-inositol, phosphatidyl-choline, phosphatidyl-éthanolamine, phosphatidyl-sérine et sphingomyeline et leurs combinaisons; où la vitamine E est un membre du group composé de pure α-, β-, γ- et δ-tocophérol, α-, β-, γ- et δ-tocotriénol et leurs esters naturelles, synthétiques et semi-synthetiques, et où l'acide gras oméga-3 est sous la forme de l'un de ses précurseurs choisis de le groupe composé d'huile de poisson, huile végétale, une combinaison de ceux-ci, une huile de poisson dérivée de toute partie d'un poisson de mer choisi d'un membre du groupe composé de saumon, de foie de morue, autres organismes marins et huile végétale choisi de le groupe composé d'huile de lin, huile de colza, huile de raisin pip et huile de microalgues et leur combinaison.

7. Composition en solution aqueuse pour l'inhibition élémentaire de l'inflammation dans l'organisme humaine et des mammifères par l'inhibition de la génération des peroxydes lipidiques pérnicieux dans les Plasma Membrane Redox System (PMRS) des cellules impliquées dans l'inflammation comprenant
a) comme ingrédient biologiquement actif une combinaison pour inhibition élémentaire de l'inflammation composée de:
i) au moins un probiotique tué et
ii) une salt ou ester de au moins une acide gras oméga-3 soluble dans l'eau et
iii) un ester soluble dans l'eau de la vitamine E naturelle semisynthetique ou synthetique, et
b) comme additifs de formulation pharmaceutiquement acceptable de l'eau et des additifs de formulation, excipients, véhicules, conservateurs et colorants soluble dans l'eau
et characterisé par les rapports de masse suivants des ingrédients:
i) d'acides gras oméga-3 : vitamine E = 100:1 à 100;
ii) d'acides gras oméga 3: probiotiques = 100:0,01 à 100.

8. Une composition selon une des revendications quelquonques de 1 à 4 comprenant en outre des excipients compatibles sur le plan pharmacologique sélectionnés du groupe composé des excipients pour l'application parentérale (intraveineuse, intramusculaire, intradermique, injection, intraoculaire, intralésionnelle, sous-cutanée), pour l'application entérale (par voie orale, transrectale) ou application nasale, ou subcutanée, pour des médicaments topiques directs sélectionnés du groupe gouttes ophthalmiques, gelées, pulvérisations, onguents, lotions, pour la livraison de liposomes encapsulées et pour des gelées tendres.

9. Une composition selon une des revendications quelquonques de 1 à 8 pour la production d'un médicament pour prévenir, traiter et atténuation l'inflammation qui peut être administré par voie parentérale, en perfusion endoveineuse, en une injection, injection sous-cutanée, intramusculaire, ou pour l'emploi intradermique, utilisation intra-hystiocytes, emploi para-hystiocytes, emploi intraarticulaire, emploi intraoculaire, ou pour administration par voi entérale, orale, transrectale et topique dans des gouttes ophthalmiques, gelées, pulvérisations, onguents, lotions, et dans des liposomes

10. Une composition selon une des revendications quelquonques de 1 à 8 pour la production d'un médicament pour prévenir, traiter et atténuation des inflammations
a) liées à des infections bactériennes, virales et fongiques;
b) induites par les vaccines courantes sélectionnées du groupe composé de l'influenza, virus de l'hépatite, BCG, la poliomyélite, Di-Per-Te (diphtérie-coqueluche-tétanos), parotitis épidémique, la rougeole et des vaccins antiallergiques,
c) une membrane muqueuse sélectionnée des membres du groupe composé de conjonctivitis, periodotitis, oesophagite, reflux gastro-oesophagien, gastrite, entérite, colite, cholécystite, cystitis, se-néphrite, sinusite, bronchite, vaginitis, prostatitis et des maladies du groupe composé de hepatitis, cirrhosis, néphritis, pleuritis et la fibrose cystique,
d) rheumatoid arthritis, diabetes juvénile (type I), maladie de Crohn, colitis ulcerosa, psoriasis, lupus erythematique et sclerosis multiplex, et
e) les maladies neuropsychiatriques, choisi du groupe composé de la schizophrénie, de dépression, d'anxiété et de panique-maladie,
f) l'inflammation allergène choisi de le groupe composé de l'asthme bronchique, dermatite atopique, fièvre des foins, conjonctivite allergique, rhinite allergique,
g) le syndrome métabolique (dyslipidémie, hypertension artérielle, diabète de type 2, obésité, stéatose hépatique),
h) maladies prolifératives néovasculaire choisis du groupe composé de retinopathy proliférative, rétinopathie du prématuré (RP) et tumeurs malignes,
i) les maladies dégénératives liées à l'âge choisis parmi le groupe composé de la maladie d'Alzheimer, la maladie Parkinson, sclérose latérale amyotrophique, l'otospongiose, l'ostéoporose, l'arthrose, sarcopenia, pilosité et les changements liés à l'âge de la peau,
j) lésions des tissus mous causés par l'inflammation post-opératoire, de blessures, activités extrêmes de sport, contusions, brûlures, congélations,
k) néoplasie, les tumeurs bénignes et malignes de la prostate et de l'utérus, cancer du sein, cancer du poumon, cancer du côlon et lymphomes, ainsi que pour le traitement des inflammations associées avec la chimiothérapie,
l) maladies ophthalmiques, uvéite, rétinopathie diabétique, la dégénérescence maculaire liée à l'âge, glaucome, cataracte,
m) l'hypertension artérielle et l'athérosclérose et des maladies liées du groupe composé de la maladie coronarienne, arythmie cardiaque, insuffisance cardiaque chronique, syndrome de néphrose, ischémie-reperfusion, maladies vasculaires périphériques, une ischémie cérébrale aiguë, ischémie cérébrale chronique et coup de sang.

11. Une composition selon une des revendications quelquonques de 1 à 8 pour la production d'un médicament pour prévenir les maladies suivantes causées par l'inflammation élémentaire: l'hypertension artérielle et l'athérosclérose et maladies connexes, maladies de l'artère coronaire, l'arythmie cardiaque, insuffisance cardiaque chronique, syndrome de néphrose, ischémie-reperfusion, maladies vasculaires périphériques, une ischémie cérébrale aiguë, ischémie cérébrale chronique et accident vasculaire cérébral.

12. Une composition selon une des revendications quelquonques de 1 à 8 pour la production d'un médicament pour prévenir, traiter, atténuer l'ischémie, l'ischémie-reperfusion, ischémie cérébrale aiguë et chronique.

13. Emploi de compositions selon une des revendication quelquonques de 1 à 8
a) pour la préparation d'un adjuvant métabolique de transfer de gène DNA et RNA naturels, semi-synthétiques et synthétiques, et
b) pour la préparation d'un adjuvant métabolique à un milieu de culture pour la culture cellulaire, culture de tissus et de culture bactérienne, et
c) pour la préparation d'un adjuvant métabolique à la survie des organes et des cellules souches transplantées.

14. Emploi d'une composition selon une des revendications quelquonques de 1 à 8 pour la préparation des extrats de tissus, de cellule, des compléments alimentaires, des aliments et nutriments pour une application humaine et vétérinaire - enrichies de oméga-3 acides gras et de vitamine E.

15. Une composition selon une des revendications quelquonques de 1 à 8 contenant de plus une substance compatible par voi pharmaceutique d'une activité biologique choisie du group composé de vitamine A, B, C, D, F, K, les stéroïdes sexuels, un metal du group sodium, calcium, magnésium, potassium, zinc; amino-carnitine, acide alpha-lipoïque, glutathion, les acides aminés essentiels, l'alcaloïde berbérine, glycosamino-glycanes du group l'acide hyaluronique, sulfate de chondroïtine, héparine, sulfate de héparine.
